# EUROPEAN PATENT APPLICATION

(11) **EP 4 483 865 A1**
(43) Date of publication of application: **01.01.2025**
(21) Application number: 22928964.0
(22) Date of filing: 22.12.2022
(51) Int. Cl.: A61K 9/70, A61K 31/196, A61K 47/04, A61K 47/38, A61P 29/00

(54) **POULTICE AND METHOD FOR MANUFACTURING SAME**

(30) Priority: 24.02.2022 JP 2022027058
(71) Applicant: Hisamitsu Pharmaceutical Co., Inc., Tosu-shi, Saga 841-0017 (JP)
(72) Inventor: SATO Sho, Tosu-shi, Saga 841-0017 (JP); TSURUSHIMA Keiichiro, Tosu-shi, Saga 841-0017 (JP); YAMASOTO Shinji, Tosu-shi, Saga 841-0017 (JP); TATEISHI Tetsuro, Tosu-shi, Saga 841-0017 (JP)
(74) Representative: Grünecker Patent- und Rechtsanwälte PartG mbB
(86) International application number: PCT/JP2022/047428
(87) International publication number: WO 2023/162447

(57) **Abstract**

The present invention relates to a poultice including: a backing layer; and an adhesive agent layer, wherein the adhesive agent layer comprises at least one selected from the group consisting of carboxymethyl cellulose and pharmaceutically acceptable salts thereof; a silicic anhydride; a drug; and a water-soluble polymer; and a particle diameter D₅₀ at which a cumulative volume in a particle size distribution of the silicic anhydride becomes 50% is 0.2 to 9.0 pm.

## Description

### [Technical Field]

The present invention relates to a poultice and a production method thereof (a method for manufacturing same).

### [Background Art]

A poultice is a patch having an adhesive agent layer comprising a water-soluble polymer and water, and is widely used as a preparation that can be expected to have a cooling effect and a warm feeling stimulation effect in addition to a medicinal effect by a drug.

Therefore, as the poultice, for example, a poultice comprising a drug having an anti-inflammatory effect, such as diclofenac, loxoprofen, and indomethacin as a drug is mainly known. As such a poultice, for example, Japanese Unexamined Patent Application Publication No. Hei. 9-208462 (Patent Literature 1) describes a self-adhesive poultice in which polybutene and gelatin are added to a poultice obtained by blending diclofenac sodium, water, and a fatty acid dialkylolamide into a poultice base.

In addition, various other poultices have been developed, for example, Japanese Unexamined Patent Application Publication No. Hei. 4-29927 (Patent Literature 2) describes a patch in which an adhesive agent layer comprises an adhesive base, a compound having a plasticizing effect on the adhesive base, silicic anhydride, a drug, and a water-absorbing filler. Furthermore, for example, Japanese Unexamined Patent Application Publication No. Hei. 6-145049 (Patent Literature 3) describes a poultice in which the silicic anhydride is blended in a crosslinked poultice base mainly composed of polyacrylic acid and/or a polyacrylate.

### [Citation List]

### [Patent Literature]

[PTL 1] Japanese Unexamined Patent Application Publication No. Hei. 9-208462
[PTL 2] Japanese Unexamined Patent Application Publication No. Hei. 4-29927
[PTL 3] Japanese Unexamined Patent Application Publication No. Hei. 6-145049

### [Summary of Invention]

### [Technical Problem]

Since a poultice comprises water as described above, it generally has a drawback that it is inferior in adhesiveness to the skin compared to a non-aqueous tape agent and the like, and easily peels off during application. Therefore, when the present inventors further studied the adhesiveness of the poultice to the skin, they found that when attempting to improve the adhesiveness to the skin, a phenomenon in which the adhesive agent layer, a part of the adhesive agent layer, or a part of the components comprised in the adhesive agent layer remains on the skin when peeled off from the skin, that is, "stickiness" may occur.

Furthermore, it is known that the adhesive agent layer of a poultice comprises an inorganic filler for the purpose of improving the shape retention thereof. As such an inorganic filler, from the viewpoint of excellent uniformity when mixed with other components, conventionally, for example, silicic anhydride having a small particle diameter and bulk density, such as Aerosil A-200 (manufactured by Nippon Aerosil Co., Ltd.) described in Patent Literature 2, has been preferably used. However, since the inorganic filler having such a small particle diameter and bulk density is difficult to handle during production due to issues such as easy generation of dust, the present inventors further studied the inorganic filler for poultices and as a result, they found problems such as the formation of aggregates during production, making it difficult to obtain an adhesive agent layer with a uniform appearance, depending on the type and particle diameter of the inorganic filler. Furthermore, they found that depending on the type and particle diameter of the inorganic filler, there may be problems that the adhesiveness to the skin may decrease, or stickiness may occur.

The present invention has been made in view of the above-described problems of the related art, and it is an object of the present invention to provide a poultice that is excellent in adhesiveness to the skin, sufficiently suppresses stickiness after peeling, and has an excellent appearance of the adhesive agent layer, and a poultice production method by which the poultice can be easily obtained.

### [Solution to Problem]

As a result of intensive studies directed to achieving the above object, the present inventors have found that in a poultice including a backing layer and an adhesive agent layer, the adhesive agent layer comprises a combination of at least one selected from the group consisting of carboxymethyl cellulose and pharmaceutically acceptable salts thereof, and silicic anhydride; and the particle diameter D₅₀ at which the cumulative volume in the particle size distribution of the silicic anhydride becomes 50% is within a specific range, thereby sufficiently improving the adhesiveness to the skin, and also sufficiently suppressing the occurrence of stickiness after peeling.

Furthermore, the present inventors have found that the combination of at least one selected from the group consisting of carboxymethyl cellulose and pharmaceutically acceptable salts thereof, and silicic anhydride can suppress the generation of dust of silicic anhydride during production as compared with the case of using silicic anhydride having a small particle diameter and bulk density, which has been conventionally preferably used, and that the formation of aggregates is sufficiently suppressed, so that an adhesive agent layer having a uniform appearance can be easily obtained, thereby completing the present invention. The aspects of the present invention obtained by such findings are as follows.
[1] A poultice including a backing layer and an adhesive agent layer,
   wherein the adhesive agent layer comprises a silicic anhydride, a drug, a water-soluble polymer, and at least one selected from the group consisting of carboxymethyl cellulose and pharmaceutically acceptable salts thereof, and
   a particle diameter D₅₀ at which a cumulative volume in a particle size distribution of the silicic anhydride becomes 50% is 0.2 to 9.0 µm.
[2] The poultice according to [1], wherein a content of the carboxymethyl cellulose and/or the pharmaceutically acceptable salt thereof is 1.0 to 4.0% by mass relative to a total mass of the adhesive agent layer in terms of sodium salt.
[3] The poultice according to [1] or [2], wherein a content of the silicic anhydride is 0.1 to 6.0% by mass relative to the total mass of the adhesive agent layer.
[4] The poultice according to any one of [1] to [3], wherein a mass ratio of the content of the carboxymethyl cellulose and/or the pharmaceutically acceptable salt thereof in terms of sodium salt to the content of the silicic anhydride (content of the carboxymethyl cellulose and/or the pharmaceutically acceptable salt thereof in terms of sodium salt : content of the silicic anhydride) in the adhesive agent layer is 1:2 to 6:0.5.
[5] The poultice according to any one of [1] to [4], wherein the drug is at least one selected from the group consisting of diclofenac, felbinac, flurbiprofen, a salicylic acid ester, indomethacin, ketoprofen, diphenhydramine, loxoprofen, lidocaine, vitamin E, glycyrrhetinic acid, capsicum extract, nonylic acid vanillylamide, l-menthol, dl-camphor, and pharmaceutically acceptable salts thereof.
[6] A method for producing a poultice including a backing layer and an adhesive agent layer,
   the method comprising the steps of:
   mixing a silicic anhydride, a drug, a water-soluble polymer, and at least one selected from the group consisting of carboxymethyl cellulose and pharmaceutically acceptable salts thereof to obtain an adhesive agent layer composition; and
   spreading the adhesive agent layer composition to obtain the adhesive agent layer, wherein
   a particle diameter D₅₀ at which a cumulative volume in a particle size distribution of the silicic anhydride becomes 50% is 0.2 to 9.0 um.
[7] The method for producing a poultice according to [6], wherein a content of the carboxymethyl cellulose and/or the pharmaceutically acceptable salt thereof in the adhesive agent layer composition and the adhesive agent layer is 1.0 to 4.0% by mass relative to a total mass of the adhesive agent layer in terms of sodium salt.
[8] The method for producing a poultice according to [6] or [7], wherein a content of the silicic anhydride in the adhesive agent layer composition and the adhesive agent layer is 0.1 to 6.0% by mass relative to the total mass of the adhesive agent layer.
[9] The method for producing a poultice according to any one of [6] to [8], wherein a mass ratio of the content of the carboxymethyl cellulose and/or the pharmaceutically acceptable salt thereof in terms of sodium salt to the content of the silicic anhydride (content of the carboxymethyl cellulose and/or the pharmaceutically acceptable salt thereof in terms of sodium salt : content of the silicic anhydride) in the adhesive agent layer composition and the adhesive agent layer is 1:2 to 6:0.5.
[10] The method for producing a poultice according to any one of [6] to [9], wherein the drug is at least one selected from the group consisting of diclofenac, felbinac, flurbiprofen, a salicylic acid ester, indomethacin, ketoprofen, diphenhydramine, loxoprofen, lidocaine, vitamin E, glycyrrhetinic acid, capsicum extract, nonylic acid vanillylamide, l-menthol, dl-camphor, and pharmaceutically acceptable salts thereof.

### [Advantageous Effects of Invention]

According to the present invention, it is possible to provide a poultice that is excellent in adhesiveness to the skin, sufficiently suppresses stickiness after peeling, and has an excellent appearance of the adhesive agent layer, and a poultice production method by which the poultice can be easily obtained.

### [Description of Embodiments]

Hereinafter, the present invention will be described in detail with reference to preferred embodiments thereof.

### <Poultice>

The poultice of the present invention is a poultice including a backing layer and an adhesive agent layer, wherein the adhesive agent layer comprises a silicic anhydride, a drug, a water-soluble polymer, and at least one selected from the group consisting of carboxymethyl cellulose and pharmaceutically acceptable salts thereof; and a particle diameter Dso at which a cumulative volume in a particle size distribution of the silicic anhydride becomes 50% is 0.2 to 9.0 µm.

The poultice of the present invention includes a backing layer. The backing layer according to the present invention is not particularly limited as long as it can support the adhesive agent layer described later, and a known backing layer for a poultice can be appropriately adopted. Examples of the material of the backing layer according to the present invention include polyolefins such as polyethylene and polypropylene; ethylene-vinyl acetate copolymers, vinyl acetate-vinyl chloride copolymers, polyvinyl chloride, and the like; polyamides such as nylon; polyesters such as polyethylene terephthalate (PET), polybutylene terephthalate, and polyethylene naphthalate; cellulose derivatives; synthetic resins such as polyurethane; and metals such as aluminum. Examples of the backing layer according to the present invention include films, sheets, fabrics, foils, and laminates thereof, among which a fabric made of fibers of the above-mentioned material is preferable, and the fabric is more preferably a woven fabric or a non-woven fabric processed by a method such as knitting, entanglement, heat-sealing, pressure-bonding, or binder-bonding of the fibers.

As such a backing layer, for example, a non-woven fabric made of polyester fibers is preferably used, and the basis weight of the non-woven fabric is more preferably 50 to 200 g/m². When the basis weight of the non-woven fabric is less than the lower limit, there is a tendency that a problem of strength, such as easy breakage when the poultice is peeled off, or a phenomenon in which components comprised in the adhesive agent layer ooze out to the back surface of the backing layer, that is, "bleeding" occurs, resulting in deterioration in the appearance and feeling in use of the poultice. On the other hand, when it exceeds the upper limit, the stretchability and flexibility of the backing layer are insufficient, so that the poultice tends to be easily peeled off.

The poultice of the present invention may further include a release liner on the surface of the adhesive agent layer opposite to the backing layer for the purpose of covering and protecting the surface of the adhesive agent layer until the poultice is used. The material of the release liner is not particularly limited, and examples thereof include polyolefins such as polyethylene and polypropylene; ethylene-vinyl acetate copolymers, vinyl acetate-vinyl chloride copolymers, polyvinyl chloride, and the like; polyamides such as nylon; polyesters such as polyethylene terephthalate; cellulose derivatives; synthetic resins such as polyurethane; aluminum; and paper. Examples of the release liner include films and sheets made of the above-mentioned materials, and laminates thereof. In addition, the release liner is preferably subjected to a release treatment, such as a silicone compound coating or a fluorine-containing compound coating, on the surface thereof in contact with the adhesive agent layer so that it can be easily peeled off from the adhesive agent layer. Among these, as the release liner, a film made of polyethylene terephthalate or polypropylene is preferable. Moreover, the thickness of such a release liner is not particularly limited, but is preferably, for example, 20 to 150 µm.

The poultice of the present invention includes an adhesive agent layer on the surface of the backing layer (usually, on one surface). The adhesive agent layer according to the present invention is not particularly limited, but the mass per unit area (area of the adhesion surface) is preferably 430 to 1900 g/m², more preferably 665 to 1500 g/m², and further preferably 900 to 1100 g/m². The area of the adhesion surface of the adhesive agent layer according to the present invention is not particularly limited because it is appropriately adjusted depending on the type and content of the drug, the purpose of treatment, the target of application, and the like, but is usually in the range of 70 to 280 cm².

### (Carboxymethyl Cellulose and Pharmaceutically Acceptable Salt Thereof)

The adhesive agent layer according to the present invention comprises at least one selected from the group consisting of carboxymethyl cellulose and pharmaceutically acceptable salts thereof (in the present specification, sometimes referred to as "carboxymethyl cellulose and/or a pharmaceutically acceptable salt thereof").

Carboxymethyl cellulose is a derivative of cellulose, also called carmellose, and has a structure in which a carboxymethyl group (-CH₂-COOH) is ether-bonded to a part of the hydroxy groups of cellulose. Theoretically, the degree of etherification is 0 to 3, but the carboxymethyl cellulose according to the present invention preferably has a degree of etherification of 0.5 to 1.5.

The carboxymethyl cellulose according to the present invention is not particularly limited, but for example, the viscosity measured at 25°C using a 1.0% aqueous solution at 60 rpm is preferably 10 to 15,000 mPa·s, more preferably 10 to 6,000 mPa-s, and further preferably 10 to 3,000 mPa·s.

In the present invention, the form of carboxymethyl cellulose in the adhesive agent layer may be a free form or a pharmaceutically acceptable salt thereof, and the pharmaceutically acceptable salt of carboxymethyl cellulose may be desalted during production and/or in the produced preparation to become a free form, and one kind of these may be used alone or two or more kinds thereof may be used in combination. Examples of the pharmaceutically acceptable salt of carboxymethyl cellulose include a sodium salt, a calcium salt, a potassium salt, and an ammonium salt, and one kind of these salts may be used alone or two or more kinds thereof may be used in combination. Among these, as the carboxymethyl cellulose comprised in the adhesive agent layer according to the present invention, a salt of carboxymethyl cellulose is preferable, and a sodium salt is more preferable.

Moreover, as the carboxymethyl cellulose and/or the pharmaceutically acceptable salt thereof, for example, commercially available products such as CMC Daicel series (manufactured by Daicel Miraizu Ltd.), Aqualon CMC series (manufactured by Ashland), Blanose CMC series (manufactured by Ashland), Cellogen series (manufactured by DKS Co., Ltd.), and TPT series (manufactured by GOTOKU CHEMICAL COMPANY LTD.) can be appropriately used.

In the present invention, the content of carboxymethyl cellulose and/or a pharmaceutically acceptable salt thereof in the adhesive agent layer (the content of carboxymethyl cellulose or the content of a pharmaceutically acceptable salt of carboxymethyl cellulose, or the total content thereof when both are comprised, the same shall apply hereinafter) is preferably 1.0 to 4.0% by mass, more preferably 1.5 to 3.5% by mass, and further preferably 2.0 to 3.0% by mass relative to the total mass of the adhesive agent layer in terms of sodium salt of carboxymethyl cellulose. If the content of carboxymethyl cellulose and/or a pharmaceutically acceptable salt thereof is less than the lower limit, stickiness after peeling from the skin tends to occur, while if it exceeds the upper limit, the adhesiveness of the adhesive agent layer to the skin tends to decrease.

### (Silicic Anhydride)

The adhesive agent layer according to the present invention comprises silicic anhydride. Silicic anhydride is also referred to as silica and is an oxide of silicon represented by SiO₂.

The particle diameter D₅₀ of the silicic anhydride according to the present invention, which is the particle diameter at which the cumulative volume in the particle size distribution becomes 50%, needs to be 0.2 to 9.0 µm, and is preferably 0.5, 1.0, 2.0, 3.0, 4.0, 4.5, or 4.8 um or more, and also preferably 5.2, 6.0, 7.0, 7.7, 8.0, or 9.0 um or less. The upper limit and lower limit of the particle diameter D₅₀ may be arbitrarily combined and are not particularly limited, but for example, 2.0 to 8.0 µm, 3.0 to 7.0 µm, 4.0 to 8.0 µm, 4.0 to 6.0 µm, 4.8 to 7.7 um, and 4.8 to 5.2 µm can be given, preferably 2.0 to 8.0 µm, more preferably 4.0 to 8.0 um, and further preferably 4.8 to 7.7 um. If the particle diameter D₅₀ of the silicic anhydride is less than the lower limit, the effect of suppressing the occurrence of stickiness after peeling the adhesive agent layer from the skin tends to decrease, or handling during production tends to be difficult, while if it exceeds the upper limit, the adhesiveness of the adhesive agent layer to the skin tends to decrease, or particles tend to remain in the adhesive agent layer, resulting in poor appearance.

In the present invention, the particle diameter D₅₀ of the silicic anhydride is measured by the laser diffraction/scattering method according to Japanese Industrial Standard JIS. Z8825:2013. More specifically, as a measurement sample, a 3 mass% aqueous dispersion of silicic anhydride to be measured is prepared, and the particle diameter in the sample is measured under the following measurement conditions:
Measuring instrument: Laser diffraction particle diameter distribution analyzer (for example, SALD-2300 manufactured by Shimadzu Corporation, high-concentration sample measurement mode)
Refractive index: 1.55
to obtain a particle size distribution curve. In the obtained particle size distribution curve, the particle diameter at which the cumulative volume becomes 50% can be defined as the particle diameter D₅₀ [pm] of silicic anhydride in the present invention. For example, when the present inventors measured the particle diameter D₅₀ of Aerosil A-200 (manufactured by Nippon Aerosil Co., Ltd.) described in Patent Literature 2 by this measurement method (more specifically, the method described in Examples below), it was 0.055 µm (n = 3, first time: 0.055 µm, second time: 0.055 um, third time: 0.055 µm).

In the present invention, the content of the silicic anhydride in the adhesive agent layer is preferably 0.1 to 6.0% by mass, more preferably 0.2 to 5.0% by mass or 0.1 to 4.0% by mass, further preferably 0.7 to 4.5% by mass, and particularly preferably 1.0 to 4.0% by mass relative to the total mass of the adhesive agent layer. If the content of silicic anhydride is outside the above range, the adhesiveness of the adhesive agent layer to the skin tends to decrease, or the effect of suppressing the occurrence of stickiness after peeling the adhesive agent layer from the skin tends to decrease.

In the present invention, the mass ratio of the content of carboxymethyl cellulose and/or a pharmaceutically acceptable salt thereof in terms of sodium salt to the content of silicic anhydride (content of carboxymethyl cellulose and/or a pharmaceutically acceptable salt thereof in terms of sodium salt : content of silicic anhydride) in the adhesive agent layer is preferably 1:2 to 6:0.5, more preferably 1:1.5 to 6:0.9, further preferably 1:1.5 to 6:1, and particularly preferably 1:1.4 to 3:1. If the content of silicic anhydride relative to the content of carboxymethyl cellulose and/or a pharmaceutically acceptable salt thereof in terms of sodium salt is less than the lower limit, the adhesiveness of the adhesive agent layer to the skin tends to decrease, or stickiness after peeling from the skin tends to occur, while if it exceeds the upper limit, the effect of suppressing the occurrence of stickiness after peeling the adhesive agent layer from the skin tends to decrease.

### (Drug)

The drug comprised in the adhesive agent layer according to the present invention is not particularly limited, and a drug that can be comprised in the adhesive agent layer of the patch can be appropriately adopted. Examples of such a drug include non-steroidal anti-inflammatory agents such as diclofenac, felbinac, flurbiprofen, salicylic acid ester, indomethacin, ketoprofen, and ibuprofen; antihistamines such as diphenhydramine and chlorpheniramine; analgesics such as aspirin, acetaminophen, and loxoprofen; local anesthetics such as lidocaine and dibucaine; muscle relaxants such as suxamethonium chloride; antifungals such as clotrimazole; antihypertensives such as clonidine; vasodilators such as nitroglycerin and isosorbide mononitrate; vitamins such as vitamin A, vitamin E, vitamin K, octotiamine, and riboflavin butyrate; prostaglandins; glycyrrhetinic acid, scopolamine, fentanyl, capsicum extract, capsaicin, nonylic acid vanillylamide, l-menthol, dl-camphor; and pharmaceutically acceptable salts thereof when they can form salts, and one kind of these may be used alone or two or more kinds thereof may be used in combination. Examples of the pharmaceutically acceptable salts include inorganic base salts such as sodium salt, potassium salt, calcium salt, and ammonium salt; organic base salts such as epolamine salt (1-(2-hydroxyethyl)pyrrolidine salt); inorganic acid salts such as hydrochloride, nitrate, and phosphate; and organic acid salts such as acetate, lactate, and benzenesulfonate.

Among these, as the drug according to the present invention, from the viewpoint of being suitable for a poultice, at least one selected from the group consisting of diclofenac, felbinac, flurbiprofen, a salicylic acid ester, indomethacin, ketoprofen, diphenhydramine, loxoprofen, lidocaine, vitamin E, glycyrrhetinic acid, capsicum extract, nonylic acid vanillylamide, l-menthol, dl-camphor, and pharmaceutically acceptable salts thereof is preferable.

For example, diclofenac is a non-steroidal analgesic and anti-inflammatory drug, and is listed in the Japanese Pharmacopoeia and other pharmacopoeias around the world. In the present invention, when diclofenac is comprised in the adhesive agent layer, the form thereof may be a free form or a pharmaceutically acceptable salt thereof, and the pharmaceutically acceptable salt of diclofenac may be desalted during production and/or in the produced preparation to become a free form, and one kind of these may be used alone or two or more kinds thereof may be used in combination. Among these, as the form of diclofenac comprised in the adhesive agent layer according to the present invention, from the viewpoint that the stability of the drug is improved, and skin irritation caused by acid and deterioration of the physical properties (such as strength, elasticity, durability, and water retention) of the adhesive agent layer tend to be further suppressed, a pharmaceutically acceptable salt of diclofenac is preferable, and a sodium salt is more preferable.

Further, for example, salicylic acid ester is also a non-steroidal analgesic and anti-inflammatory drug, and examples of the salicylic acid ester according to the present invention include methyl salicylate and glycol salicylate. Among these, as the salicylic acid ester comprised in the adhesive agent layer according to the present invention, glycol salicylate is preferable from the viewpoint of less odor.

In the present invention, the content of the drug in the adhesive agent layer (the total content thereof when two or more drugs are used, the same shall apply hereinafter) is appropriately adjusted according to the effect, purpose, and the like, and thus cannot be generally specified, but is preferably 0.2 to 10.0% by mass relative to the total mass of the adhesive agent layer.

For example, when the adhesive agent layer according to the present invention comprises at least one selected from the group consisting of diclofenac and a pharmaceutically acceptable salt thereof (hereinafter sometimes referred to as "diclofenac and/or a pharmaceutically acceptable salt thereof"), the content of diclofenac and/or a pharmaceutically acceptable salt thereof (the content of diclofenac or the content of a pharmaceutically acceptable salt of diclofenac, or the total content thereof when both are comprised, the same shall apply hereinafter) is preferably 0.5 to 3.0% by mass, more preferably 0.75 to 2.5% by mass, and further preferably 1.0 to 2.0% by mass relative to the total mass of the adhesive agent layer in terms of sodium salt of diclofenac.

Further, for example, when the adhesive agent layer according to the present invention comprises a salicylic acid ester, the content of the salicylic acid ester (the total content thereof when two or more salicylic acid esters are comprised, the same shall apply hereinafter) is preferably 0.5 to 3.0% by mass, more preferably 0.75 to 2.5% by mass, and further preferably 1.0 to 2.0% by mass relative to the total mass of the adhesive agent layer in terms of glycol salicylate.

If the content of these drugs is less than the lower limit, the analgesic and anti-inflammatory effect tends to decrease, while if it exceeds the upper limit, side effects tend to occur easily.

### (Water-Soluble Polymer)

The adhesive agent layer according to the present invention further comprises a water-soluble polymer other than carboxymethyl cellulose and/or a pharmaceutically acceptable salt thereof (sometimes simply referred to as "water-soluble polymer" in the present specification). Such a water-soluble polymer is not particularly limited, and a known water-soluble polymer conventionally comprised in the adhesive agent layer of a poultice can be appropriately adopted.

Examples of the water-soluble polymer according to the present invention (water-soluble polymer other than carboxymethyl cellulose and/or a pharmaceutically acceptable salt thereof) include polyacrylic acid neutralized product, polyacrylic acid, carboxyvinyl polymer, methyl cellulose, ethyl cellulose, hydroxyethyl cellulose, hydroxypropyl cellulose, crystalline cellulose, gelatin, casein, pullulan, agar, dextran, dextrin, sodium alginate, soluble starch, carboxylated starch, polyvinyl alcohol, polyethylene oxide, polyacrylamide, polyvinylpyrrolidone, polyvinyl ether-maleic anhydride copolymer, methoxyethylene-maleic anhydride copolymer, isobutylene-maleic anhydride copolymer, polyethyleneimine, sodium hyaluronate, guar gum, xanthan gum, gellan gum, carrageenan, locust bean gum, and pectin, and one kind of these may be used alone or two or more kinds thereof may be used in combination.

Among these, as the water-soluble polymer according to the present invention, from the viewpoint of further improving the shape retention of the adhesive agent layer, at least one selected from the group consisting of polyacrylic acid neutralized product, polyacrylic acid, gelatin, agar, sodium alginate, polyvinyl alcohol, polyvinylpyrrolidone, sodium hyaluronate, guar gum, xanthan gum, gellan gum, carrageenan, locust bean gum, and pectin is preferable, and at least polyacrylic acid neutralized product is more preferable.

In the present invention, the "polyacrylic acid neutralized product" includes a polyacrylic acid completely neutralized product and a polyacrylic acid partially neutralized product, and indicates a product in which all or part of polyacrylic acid is neutralized with an alkali metal such as sodium and potassium, ammonium ions, and the like. Examples of the polyacrylic acid neutralized product include sodium polyacrylate, potassium polyacrylate, and ammonium polyacrylate, and one kind of these may be used alone or two or more kinds thereof may be used in combination, but from the viewpoint that the shape retention of the obtained adhesive agent layer tends to be further enhanced, a polyacrylic acid partially neutralized product is preferable, and sodium polyacrylate is also preferable. The neutralization rate of the polyacrylic acid neutralized product is not particularly limited, but is preferably, for example, 30 to 100%, more preferably 35 to 65%, and further preferably 40 to 60%.

In the present invention, the content of the water-soluble polymer in the adhesive agent layer (the total content thereof when two or more water-soluble polymers are used, the same shall apply hereinafter) is preferably 5.0 to 15.0% by mass, and more preferably 8.5 to 9.0% by mass relative to the total mass of the adhesive agent layer. If the content of the water-soluble polymer is less than the lower limit, the shape retention of the adhesive agent layer tends to decrease, while if it exceeds the upper limit, the adhesiveness of the adhesive agent layer to the skin tends to decrease. Further, when the adhesive agent layer according to the present invention comprises the polyacrylic acid neutralized product, the content thereof (the total content thereof when two or more polyacrylic acid neutralized products are used, the same shall apply hereinafter) is preferably 1.0 to 10.0% by mass, more preferably 2.0 to 7.0% by mass, and further preferably 3.0 to 4.0% by mass relative to the total mass of the adhesive agent layer from the same viewpoint.

### (Water)

The adhesive agent layer according to the present invention also comprises water. The water is preferably water that has been subjected to purification such as ion exchange, distillation, and filtration, and for example, "purified water" described in the Japanese Pharmacopoeia (18th Edition of the Japanese Pharmacopoeia) can be suitably used. The content of water in the adhesive agent layer is preferably 20 to 70% by mass relative to the total mass of the adhesive agent layer. If the content of the water is outside the above range, the shape retention of the adhesive agent layer tends to decrease, or the adhesiveness to the skin tends to decrease.

### (Polyhydric Alcohol)

The adhesive agent layer according to the present invention preferably further comprises a polyhydric alcohol from the viewpoint that it functions as a humectant, and the adhesiveness to the skin tends to be further improved. Such a polyhydric alcohol is not particularly limited, and examples thereof include glycerin; alkylene glycols such as methylene glycol, ethylene glycol, propylene glycol, and butylene glycol; polyalkylene glycols such as polyethylene glycol; alkane diols such as 1,3-propanediol and 1,4-butanediol; and sorbitol, and one kind of these may be used alone or two or more kinds thereof may be used in combination.

Among these, the polyhydric alcohol is preferably at least one selected from the group consisting of glycerin and alkylene glycols, and more preferably comprises at least glycerin from the viewpoint that the adhesiveness of the adhesive agent layer to the skin tends to be further improved.

When the adhesive agent layer according to the present invention comprises the polyhydric alcohol, the content thereof (the total content thereof when two or more polyhydric alcohols are used, the same shall apply hereinafter) is preferably 10 to 50% by mass, and more preferably 35 to 45% by mass relative to the total mass of the adhesive agent layer. If the content of the polyhydric alcohol is less than the lower limit, the effect of improving the adhesiveness tends to be not sufficiently exerted, while if it exceeds the upper limit, the shape retention of the adhesive agent layer tends to decrease. Further, when the adhesive agent layer according to the present invention comprises glycerin, the content thereof is preferably 20 to 50% by mass, more preferably 25 to 43% by mass, and further preferably 30 to 35% by mass relative to the total mass of the adhesive agent layer from the same viewpoint.

### (Chelating Agent)

The adhesive agent layer according to the present invention preferably further comprises a chelating agent from the viewpoint that uneven spreading of the adhesive agent layer tends to be further suppressed. Such a chelating agent is not particularly limited, and examples thereof include edetate (EDTA), pyrophosphate, hexametaphosphate, and gluconate, and one kind of these may be used alone or two or more kinds thereof may be used in combination. Among these, as the chelating agent, sodium edetate is preferable.

When the adhesive agent layer according to the present invention comprises the chelating agent, the content thereof (the total content thereof when two or more chelating agents are used, the same shall apply hereinafter) is preferably 0.01 to 1% by mass, and more preferably 0.01 to 0.1% by mass relative to the total mass of the adhesive agent layer. If the content of the chelating agent is less than the lower limit, the effect of suppressing uneven spreading of the adhesive agent layer is not sufficiently exerted, and the appearance tends to be impaired, while if it exceeds the upper limit, the shape retention of the adhesive agent layer tends to decrease.

### (Crosslinking Agent)

The adhesive agent layer according to the present invention preferably further comprises a crosslinking agent from the viewpoint that the shape retention of the adhesive agent layer tends to be further improved, and the uneven spreading tends to be further suppressed. Such a crosslinking agent is not particularly limited, and examples thereof include aluminum potassium sulfate (alum), calcium chloride, magnesium chloride, aluminum hydroxide, and dihydroxyaluminum aminoacetate, and one kind of these may be used alone or two or more kinds thereof may be used in combination. Among these, as the crosslinking agent, aluminum potassium sulfate is preferable.

When the adhesive agent layer according to the present invention comprises the crosslinking agent, the content thereof (the total content thereof when two or more crosslinking agents are used, the same shall apply hereinafter) is preferably 0.1 to 1.0% by mass, and more preferably 0.2 to 0.5% by mass relative to the total mass of the adhesive agent layer. If the content of the crosslinking agent is less than the lower limit, the effect of improving the shape retention of the adhesive agent layer tends to be not sufficiently exerted, while if it exceeds the upper limit, the effect of suppressing uneven spreading of the adhesive agent layer is not sufficiently exerted, and the appearance tends to be impaired.

Further, when the adhesive agent layer according to the present invention comprises both the chelating agent and the crosslinking agent, the mass ratio of the content of the chelating agent to the content of the crosslinking agent (content of chelating agent:content of crosslinking agent) is preferably 1:0.7 to 1:10, and more preferably 1:4 to 1:9. If the content of the crosslinking agent relative to the content of the chelating agent is less than the lower limit, bleeding tends to occur, or the effect of improving the shape retention of the adhesive agent layer tends to be not sufficiently exerted, while if it exceeds the upper limit, the effect of suppressing uneven spreading of the adhesive agent layer is not sufficiently exerted, so that the appearance tends to be impaired, or the adhesive strength of the adhesive agent layer tends to decrease.

### (pH Adjuster)

The adhesive agent layer according to the present invention preferably further comprises a pH adjuster in order to adjust the pH thereof. Examples of such a pH adjuster include organic acids such as acetic acid, lactic acid, oxalic acid, citric acid, and tartaric acid; inorganic acids such as hydrochloric acid, sulfuric acid, nitric acid, and phosphoric acid; pharmaceutically acceptable salts of the organic acids and the inorganic acids; amines such as monoethanolamine, diethanolamine, triethanolamine, isopropanolamine, and diisopropanolamine; and salts of hydroxides such as sodium hydroxide, potassium hydroxide, and magnesium hydroxide, and one kind of these may be used alone or two or more kinds thereof may be used in combination. The pH of the adhesive agent layer according to the present invention is preferably 7.0 to 9.0, and more preferably 7.0 to 8.5.

### (Tackifier)

The adhesive agent layer according to the present invention preferably further comprises a tackifier from the viewpoint that the adhesiveness of the adhesive agent layer to the skin tends to be further improved. Such a tackifier is not particularly limited, and examples thereof include acrylic acid ester copolymers such as aminoalkyl methacrylate copolymer E; and methyl acrylate-2-ethylhexyl acrylate copolymer, and one kind of these may be used alone or two or more kinds thereof may be used in combination. Among these, as the tackifier, aminoalkyl methacrylate copolymer E is preferable from the viewpoint that the effect tends to be exerted even with a small amount of addition.

When the adhesive agent layer according to the present invention comprises the tackifier, the content thereof (the total content thereof when two or more tackifiers are used, the same shall apply hereinafter) is preferably 0.1 to 1.0% by mass, and more preferably 0.4 to 0.6% by mass relative to the total mass of the adhesive agent layer. If the content of the tackifier is less than the lower limit, the effect of improving the adhesiveness of the adhesive agent layer to the skin tends to be not sufficiently exerted, while if it exceeds the upper limit, pain tends to occur when the adhesive agent layer is peeled off from the skin.

### (Other Additives)

The adhesive agent layer according to the present invention may further comprise other additives, such as an emulsifier, a refreshing agent, a stabilizer (antioxidant), a preservative, and a filler other than the silicic anhydride according to the present invention.

Examples of the emulsifier include polyalkylene glycol monooleates (such as polyethylene glycol monooleate, polypropylene glycol monooleate, and polyethylene polypropylene glycol monooleate), polyethylene glycol monostearate, and polyoxyethylene sorbitan monooleate, and one kind of these may be used alone or two or more kinds thereof may be used in combination.

Examples of the refreshing agent include thymol, dl-menthol, 1-isopulegol, and peppermint oil, and one kind of these may be used alone or two or more kinds thereof may be used in combination.

Examples of the stabilizer include sodium sulfite, butylhydroxyanisole, dibutylhydroxytoluene, nordihydroguaiaretic acid, citric acid, ascorbic acid, and propyl gallate, and one kind of these may be used alone or two or more kinds thereof may be used in combination.

Examples of the preservative include methyl para-hydroxybenzoate, ethyl para-hydroxybenzoate, propyl para-hydroxybenzoate, butyl para-hydroxybenzoate, 1,2-pentanediol, benzoic acid and salts thereof, salicylic acid and salts thereof, sorbic acid and salts thereof, dehydroacetic acid and salts thereof, 4-isopropyl-3-methylphenol, 2-isopropyl-5-methylphenol, phenol, hinokitiol, cresol, 2,4,4'-trichloro-2'-hydroxydiphenyl ether, 3,4,4'-trichlorocarbanilide, chlorobutanol, benzalkonium chloride, and benzethonium chloride, and one kind of these may be used alone or two or more kinds thereof may be used in combination.

Examples of the filler other than the silicic anhydride according to the present invention include silicic anhydride having a particle diameter outside the range of the silicic anhydride according to the present invention, and inorganic fillers such as titanium oxide, aluminum silicate, kaolin, talc, bentonite, and magnesium aluminum metasilicate, and one kind of these may be used alone or two or more kinds thereof may be used in combination. However, the adhesive agent layer according to the present invention preferably does not substantially comprise a filler other than the silicic anhydride according to the present invention, and even when it is comprised, the content thereof (the total content thereof when two or more fillers are used, the same shall apply hereinafter) is preferably 3.0% by mass or less, more preferably 1.0% by mass or less, further preferably 0.1% by mass or less, and particularly preferably 0.05% by mass or less relative to the total mass of the adhesive agent layer.

### <Poultice Production Method>

The poultice of the present invention is not particularly limited, and can be produced by appropriately adopting a known poultice production method, but is preferably produced by the following production method:
A method for producing a poultice including a backing layer and an adhesive agent layer,
the method including the steps of:
   mixing the silicic anhydride, the drug, and the water-soluble polymer, and at least one selected from the group consisting of the carboxymethyl cellulose and the pharmaceutically acceptable salts thereof to obtain an adhesive agent layer composition; and
   spreading the adhesive agent layer composition to obtain the adhesive agent layer.

In the step of obtaining the adhesive agent layer composition, the carboxymethyl cellulose and/or a pharmaceutically acceptable salt thereof, the silicic anhydride, the drug, the water-soluble polymer, water, and, if necessary, the polyhydric alcohol, the chelating agent, the crosslinking agent, the pH adjuster, the tackifier, the additive, and the like are mixed to obtain an adhesive agent layer composition. At this time, the blending amount of each component to be mixed is as described above as the content of each component in the adhesive agent layer of the poultice of the present invention, including the preferred embodiments thereof.

The mixing method is not particularly limited, and a known method in conventional methods for producing poultices or a method analogous thereto can be appropriately adopted, and examples thereof include a method of mixing using a mixer such as a propeller mixer, a paddle mixer, an anchor mixer, a planetary mixer, a V-type mixer, and a Henschel mixer. The mixing conditions are also not particularly limited, and can be appropriately adjusted to conventionally known conditions or conditions analogous thereto.

Next, in the step of obtaining the adhesive agent layer, the adhesive agent layer composition obtained above is spread to obtain the adhesive agent layer. The spreading method is not particularly limited, and a known method in conventional methods for producing poultices or a method analogous thereto can be appropriately adopted, and for example, the adhesive agent layer composition is spread on the surface of the backing layer (usually, on one surface) to a predetermined thickness to obtain the poultice of the present invention in which the adhesive agent layer is provided on the backing layer. In this case, if necessary, the release liner may be bonded to the surface of the adhesive agent layer opposite to the backing layer to obtain a poultice in which the backing layer, the adhesive agent layer, and the release liner are laminated in this order.

As another aspect of the spreading method, for example, after the adhesive agent layer composition is first spread on one surface of the release liner to a predetermined thickness to obtain the adhesive agent layer, the backing layer is bonded to the surface of the adhesive agent layer opposite to the release liner to obtain a poultice in which the backing layer, the adhesive agent layer, and the release liner are laminated in this order.

Furthermore, the method for producing a poultice of the present invention may further include a step of cutting the poultice into a predetermined shape, if necessary.

Moreover, the poultice of the present invention may be enclosed in a packaging container for storage (for example, an aluminum packaging bag) to form a packaged product, if necessary.

### [Examples]

Hereinafter, the present invention will be described more specifically based on Examples and Comparative Examples, but the present invention is not limited to the following Examples. In each of Examples and Comparative Examples, the particle diameter measurement of silicic anhydride or magnesium aluminum metasilicate used, and the adhesiveness evaluation, stickiness evaluation, and adhesive agent layer appearance evaluation of the poultice obtained were performed by the methods shown below, respectively.

### (Particle Diameter Measurement)

The particle diameter of silicic anhydride or magnesium aluminum metasilicate was measured by the laser diffraction/scattering method according to Japanese Industrial Standard JIS. Z8825:2013 under the following measurement conditions:
Measuring instrument: Laser diffraction particle diameter distribution analyzer SALD-2300 manufactured by Shimadzu Corporation
Measurement mode: High-concentration sample measurement mode
Sample concentration: 3% by mass
Dispersion medium: Purified water
Refractive index: 1.55

The particle diameter in the sample was measured to obtain a particle size distribution curve. From the obtained particle size distribution curve, the particle diameter D₅₀ (pm) at which the cumulative volume becomes 50% was determined. The measurement was performed three times for each silicic anhydride sample or magnesium aluminum metasilicate sample, and the average was taken as the particle diameter (particle diameter D₅₀ [µm]) of the silicic anhydride or magnesium aluminum metasilicate.

### (Adhesiveness Evaluation)

First, six poultices were prepared according to the same Example or Comparative Example, the release liner was peeled off from each poultice, and each poultice was applied to the elbow of each of six subjects. Next, the subject's arm was bent and stretched (flexed and extended), and the peeling state of the adhesive agent layer from the skin was observed after bending and stretching 40 times. The peeling state was scored based on the criteria in Table 1 below, and the adhesiveness was evaluated from the average value (average score) of the scores of the six subjects. The adhesiveness evaluation of the poultice was judged to be "B" or higher, which means that the adhesiveness to the skin is sufficiently acceptable as a preparation.

**[Table 1]**

| Peeling State | Score | Avg. Score: Evaluation |
|---|---|---|
| No Peeling | 100 | 80 to 100: A |
| Edge Peeling (peeling of less than 1/4 of the adhesion surface) | 80 | |
| Peeling of 1/4 to less than 113 of the adhesion surface | 60 | |
| | | 65 to less than 80: B |
| Peeling of 1/3 to less than 1/2 of the adhesion surface | 40 | |
| | | Less than 65: C |
| Peeling of 1/2 or more of the adhesion surface | 20 | |
| Falling Off | 0 | |

### (Stickiness Evaluation)

After the above adhesiveness evaluation, each poultice was peeled off from the skin, and the stickiness condition was observed when the site where the poultice had been applied was touched with a fingertip. The stickiness condition was scored based on the criteria in Table 2 below, and the stickiness was evaluated from the average value (average score) of the scores of the six subjects. The stickiness evaluation of the poultice was judged to be "B" or higher, which means that the occurrence of stickiness to the skin is suppressed to a sufficiently acceptable level as a preparation.

**[Table 2]**

| Stickiness Condition | Score | Avg. Score: Evaluation |
|---|---|---|
| Not Sticky | 100 | 70 to 100: A |
| Almost Not Sticky (no problem in use) | 75 | |
| Slightly Sticky (within acceptable range) | 50 | |
| | | 50 to less than 70: B |
| | | Less than 50: C |
| Sticky (limit of acceptability) | 25 | |
| Very Sticky (outside acceptable range) | 0 | |

### (Adhesive Agent Layer Appearance Evaluation)

The release liner was peeled off from the poultice immediately after it was obtained in Example or Comparative Example, and the surface of the adhesive agent layer was visually observed. A poultice in which aggregates or unevenness was confirmed on the surface of the adhesive agent layer and use as a preparation was difficult was evaluated as "N," and a poultice in which no aggregates or unevenness was confirmed on the surface of the adhesive agent layer and the surface was uniform was evaluated as "Y."

### (Example 1)

First, 2.0 parts by mass of sodium carboxymethyl cellulose (degree of etherification: 0.8 to 1.0, viscosity (25°C, 1.0% aqueous solution, 60 rpm): 13 mPa·s), 1.5 parts by mass of silicic anhydride, 1.0 part by mass of diclofenac sodium, 8.8 parts by mass of a water-soluble polymer (gelatin : polyacrylic acid partially neutralized product (sodium polyacrylate) : polyvinyl alcohol = 1.8:3.5:3.5 (mass ratio)), 39.2 parts by mass of a polyhydric alcohol (glycerin : alkylene glycol = 31.6:7.6 (mass ratio)), 0.05 part by mass of a chelating agent (sodium edetate hydrate), 0.41 part by mass of a crosslinking agent (dried aluminum potassium sulfate), 1.9 parts by mass of a pH adjuster (diethanolamine), 0.5 part by mass of a tackifier (aminoalkyl methacrylate copolymer E), 2.72 parts by mass of other additives (an emulsifier, a refreshing agent, and a stabilizer), and 41.92 parts by mass of purified water were mixed to prepare an adhesive agent layer composition. The particle diameter of the silicic anhydride measured by the above particle diameter measurement was as follows: particle diameter Dso: 4.8 um.

Next, the obtained adhesive agent layer composition was uniformly spread on a release liner (polypropylene film) so as to be 14 g per poultice (140 mm × 100 mm), and a backing layer (polyester non-woven fabric, basis weight: 100 g/m²) was laminated thereon to obtain a poultice. The composition of the obtained adhesive agent layer is shown in Table 3 below. In addition, the results of the adhesiveness evaluation, stickiness evaluation, and adhesive agent layer appearance evaluation of the obtained poultice are also shown in Table 3 below.

### (Example 2, Comparative Examples 1 to 6)

Each poultice was obtained in the same manner as in Example 1, except that the composition of the adhesive agent layer was changed to the compositions shown in Table 3 below, respectively. The results of the adhesiveness evaluation, stickiness evaluation, and adhesive agent layer appearance evaluation of the obtained poultices are shown in Table 3 below, together with the composition of each adhesive agent layer.

**[Table 3]**

| | | Comparative Example 1 | Comparative Example 2 | Comparative Example 3 | Example 1 | Example 2 | Comparative Example 4 | Comparative Example 5 | Comparative Example 6 |
|---|---|---|---|---|---|---|---|---|---|
| Sodium Carboxymethyl Cellulose | | - | - | 3.0 | 2.0 | 3.0 | - | - | - |
| Hydroxypropyl Cellulose | | - | - | - | - | - | 3.0 | - | - |
| Carboxyvinyl Polymer | | - | - | - | - | - | - | 3.0 | -- |
| Crystalline Cellulose | | - | - | - | - | - | - | - | 3.0 |
| Silicic Anhydride (Particle Diameter D50: 4.8 µm) | | - | 1.5 | - | 1.5 | 1.0 | 1.5 | 1.5 | 1.5 |
| Diclofenac Sodium | | 1.0 | 1.0 | 1.0 | 1.0 | 1.0 | 1.0 | 1.0 | 1.0 |
| Water-Soluble Polymer | | 8.8 | 8.8 | 8.8 | 8.8 | 8.8 | 8.8 | 8.8 | 8.8 |
| Polyhydric Alcohol | | 39.2 | 39.2 | 39.2 | 39.2 | 39.2 | 39.2 | 39.2 | 39.2 |
| Chelating Agent | | 0.05 | 0.05 | 0.05 | 0.05 | 0.05 | 0.05 | 0.05 | 0.05 |
| Crosslinking Agent | | 0.41 | 0.41 | 0.41 | 0.41 | 0.41 | 0.41 | 0.41 | 0.41 |
| pH Adjuster | | 1.9 | 1.9 | 1.9 | 1.9 | 1.9 | 1.9 | 1.9 | 1.9 |
| Tackifier | | 0.5 | 0.5 | 0.5 | 0.5 | 0.5 | 0.5 | 0.5 | 0.5 |
| Others | | 2.72 | 2.72 | 2.72 | 2.72 | 2.72 | 2.72 | 2.72 | 2.72 |
| Purified Water | | 45.42 | 43.92 | 42.42 | 41.92 | 41.42 | 40.92 | 40.92 | 40.92 |
| Total (parts by mass) | | 100 | 100 | 100 | 100 | 100 | 100 | 100 | 100 |
| Adhesiveness | Evaluation | C | A | C | B | A | B | C | B |
| | Avg. Score | 53.3 | 97.1 | 56.7 | 73.3 | 83.3 | 70 | 60 | 66.7 |
| Stickiness | Evaluation | C | C | B | B | A | C | A | C |
| | Avg. Score | 45.8 | 39.3 | 66.7 | 54.2 | 73.3 | 45.8 | 79.2 | 45.8 |
| Adhesive Agent Layer Appearance Evaluation | | Y | Y | Y | Y | Y | Y | N | Y |

As is clear from the results shown in Table 3, in the poultice of the present invention (for example, Examples 1 and 2), the adhesiveness evaluation, the stickiness evaluation, and the adhesive agent layer appearance evaluation were all confirmed to be sufficiently excellent as a poultice by comprising both silicic anhydride and sodium carboxymethyl cellulose. On the other hand, in the poultice not comprising silicic anhydride or at least one of carboxymethyl cellulose and/or a pharmaceutically acceptable salt thereof (for example, Comparative Examples 1 to 3), and the poultice comprising hydroxypropyl cellulose, carboxyvinyl polymer, or crystalline cellulose instead of carboxymethyl cellulose and/or a pharmaceutically acceptable salt thereof (for example, Comparative Examples 4 to 6), at least one of the adhesiveness evaluation, the stickiness evaluation, and the adhesive agent layer appearance evaluation was inferior, and it was confirmed that use as a poultice was difficult.

### (Examples 3 and 4)

Each poultice was obtained in the same manner as in Example 1, except that the composition of the adhesive agent layer was changed to the compositions shown in Table 4 below, respectively.

### (Comparative Examples 7 to 9)

Each poultice was obtained in the same manner as in Example 1, except that silicic anhydride or magnesium aluminum metasilicate having a particle diameter D₅₀ shown in Table 4 below was used instead of silicic anhydride having a particle diameter D₅₀: 4.8 um, and the composition of the adhesive agent layer was changed to the compositions shown in Table 4 below, respectively. The particle diameter D₅₀ of silicic anhydride or magnesium aluminum metasilicate was measured by the above particle diameter measurement, respectively.

### (Example 5)

A poultice was obtained in the same manner as in Example 1, except that silicic anhydride having a particle diameter D₅₀ of 2.2 µm was used instead of silicic anhydride having a particle diameter D₅₀: 4.8 um, and the composition of the adhesive agent layer was changed to the composition shown in Table 4 below. The particle diameter D₅₀ of silicic anhydride was measured by the above particle diameter measurement.

### (Example 6)

A poultice was obtained in the same manner as in Example 1, except that silicic anhydride having a particle diameter D₅₀ of 3.1 µm was used instead of silicic anhydride having a particle diameter D₅₀: 4.8 um, glycol salicylate was used instead of diclofenac sodium, and the composition of the adhesive agent layer was changed to the composition shown in Table 4 below. The particle diameter D₅₀ of silicic anhydride was measured by the above particle diameter measurement.

### (Example 7)

A poultice was obtained in the same manner as in Example 1, except that silicic anhydride having a particle diameter D₅₀ of 7.7 µm was used instead of silicic anhydride having a particle diameter D₅₀: 4.8 um, and the composition of the adhesive agent layer was changed to the composition shown in Table 4 below. The particle diameter D₅₀ of silicic anhydride was measured by the above particle diameter measurement.

The results of the adhesiveness evaluation, stickiness evaluation, and adhesive agent layer appearance evaluation of the poultices obtained in Examples 3 to 7 and Comparative Examples 7 to 9 are shown in Table 4 below, together with the composition of each adhesive agent layer. In Comparative Example 9, since the adhesive agent layer appearance evaluation result was N and use as a preparation was difficult, the adhesiveness evaluation and stickiness evaluation were not performed. Table 4 also shows the results and the composition of the adhesive agent layer of Example 2.

**[Table 4]**

| | | Comparative Example 7 | Example 5 | Example 6 | Example 3 | Example 2 | Example 4 | Example 7 | Comparative Example 8 | Comparative Example 9 |
|---|---|---|---|---|---|---|---|---|---|---|
| Sodium Carboxymethyl Cellulose | | 3.0 | 3.0 | 3.0 | 3.0 | 3.0 | 3.0 | 3.0 | 3.0 | 3.0 |
| Silicic Anhydride | | 3.0 | - | - | - | - | - | - | - | - |
| (Particle Diameter D50: 0.12 µm) | | | | | | | | | | |
| Silicic Anhydride | | - | 3.0 | - | - | - | - | - | - | - |
| (Particle Diameter D50: 2.2 µm) | | | | | | | | | | |
| Silicic Anhydride | | - | - | 3.0 | - | - | - | - | - | - |
| (Particle Diameter D50: 3.1 pm) | | | | | | | | | | |
| Silicic Anhydride | | - | - | - | 0.5 | 1.0 | 3.0 | - | - | - |
| (Particle Diameter D50: 4.8 µm) | | | | | | | | | | |
| Silicic Anhydride | | - | - | - | - | - | - | 4.0 | - | - |
| (Particle Diameter D50: 7.7 µm) | | | | | | | | | | |
| Silicic Anhydride | | - | - | - | - | - | - | - | 3.0 | - |
| (Particle Diameter D50: 9.6 µm) | | | | | | | | | | |
| Magnesium Aluminum Metasilicate | | - | - | - | - | - | - | - | - | 3.0 |
| (Particle Diameter D50: 3.8 µm) | | | | | | | | | | |
| Diclofenac Sodium | | 1.0 | 1.0 | - | 1.0 | 1.0 | 1.0 | 1.0 | 1.0 | 1.0 |
| Glycol Salicylate | | - | - | 1.0 | - | - | - | - | - | - |
| Water-Soluble Polymer | | 8.8 | 8.8 | 8.8 | 8.8 | 8.8 | 8.8 | 8.8 | 8.8 | 8.8 |
| Polyhydric Alcohol | | 39.2 | 39.2 | 39.2 | 39.2 | 39.2 | 39.2 | 39.2 | 39.2 | 39.2 |
| Chelating Agent | | 0.05 | 0.05 | 0.05 | 0.05 | 0.05 | 0.05 | 0.05 | 0.05 | 0.05 |
| Crosslinking Agent | | 0.41 | 0.41 | 0.41 | 0.41 | 0.41 | 0.41 | 0.41 | 0.41 | 0.41 |
| pH Adjuster | | 1.9 | 1.9 | 1.9 | 1.9 | 1.9 | 1.9 | 1.9 | 1.9 | 1.9 |
| Tackifier | | 0.5 | 0.5 | 0.5 | 0.5 | 0.5 | 0.5 | 0.5 | 0.5 | 0.5 |
| Others | | 2.72 | 2.72 | 2.72 | 2.72 | 2.72 | 2.72 | 2.72 | 2.72 | 2.72 |
| Purified Water | | 39.42 | 39.42 | 39.42 | 41.92 | 41.42 | 39.42 | 38.42 | 39.42 | 39.42 |
| Total (parts by mass) | | 100 | 100 | 100 | 100 | 100 | 100 | 100 | 100 | 100 |
| Adhesiveness | Evaluation | A | A | A | B | A | A | A | C | - |
| | Avg. Score | 86.7 | 96.7 | 96.7 | 76.7 | 83.3 | 86.7 | 93.3 | 56.7 | |
| Stickiness | Evaluation | C | B | B | A | A | A | A | A | - |
| | Avg. Score | 41.7 | 66.7 | 62.5 | 75 | 73.3 | 70.8 | 70.8 | 83.3 | |
| Adhesive Agent Layer Appearance Evaluation | | Y | Y | Y | Y | Y | Y | Y | Y | N |

As is clear from the results shown in Table 4, the poultice of the present invention comprises both silicic anhydride and carboxymethyl cellulose and/or a pharmaceutically acceptable salt thereof, and the particle diameter D₅₀ of the silicic anhydride is within a specific range (for example, 2.0 to 8.0 um), so that the adhesiveness evaluation, the stickiness evaluation, and the adhesive agent layer appearance evaluation were all confirmed to be sufficiently excellent as a poultice. On the other hand, in the poultice in which the particle diameter of silicic anhydride was outside the above range (for example, Comparative Examples 7 and 8) and the poultice in which magnesium aluminum metasilicate was used instead of silicic anhydride (for example, Comparative Example 9), at least one of the adhesiveness evaluation, the stickiness evaluation, and the adhesive agent layer appearance evaluation was inferior, and it was confirmed that use as a poultice was difficult.

### [Industrial Applicability]

As described above, according to the present invention, it is possible to provide a poultice that is excellent in adhesiveness to the skin, sufficiently suppresses stickiness after peeling, and has an excellent appearance of the adhesive agent layer, and a poultice production method by which the poultice can be easily obtained.

## Claims

1. A poultice including a backing layer and an adhesive agent layer,
wherein the adhesive agent layer comprises a silicic anhydride, a drug, a water-soluble polymer, and at least one selected from the group consisting of carboxymethyl cellulose and pharmaceutically acceptable salts thereof, and
a particle diameter Dso at which a cumulative volume in a particle size distribution of the silicic anhydride becomes 50% is 0.2 to 9.0 µm.

2. The poultice according to claim 1, wherein a content of the carboxymethyl cellulose and/or the pharmaceutically acceptable salt thereof is 1.0 to 4.0% by mass relative to a total mass of the adhesive agent layer in terms of sodium salt.

3. The poultice according to claim 1 or 2, wherein a content of the silicic anhydride is 0.1 to 6.0% by mass relative to the total mass of the adhesive agent layer.

4. The poultice according to claim 1 or 2, wherein a mass ratio of the content of the carboxymethyl cellulose and/or the pharmaceutically acceptable salt thereof in terms of sodium salt to the content of the silicic anhydride (content of the carboxymethyl cellulose and/or the pharmaceutically acceptable salt thereof in terms of sodium salt : content of the silicic anhydride) in the adhesive agent layer is 1:2 to 6:0.5.

5. The poultice according to claim 1 or 2, wherein the drug is at least one selected from the group consisting of diclofenac, felbinac, flurbiprofen, a salicylic acid ester, indomethacin, ketoprofen, diphenhydramine, loxoprofen, lidocaine, vitamin E, glycyrrhetinic acid, capsicum extract, nonylic acid vanillylamide, l-menthol, dl-camphor, and pharmaceutically acceptable salts thereof.

6. A method for producing a poultice including a backing layer and an adhesive agent layer,
the method comprising the steps of:
mixing a silicic anhydride, a drug, a water-soluble polymer, and at least one selected from the group consisting of carboxymethyl cellulose and pharmaceutically acceptable salts thereof to obtain an adhesive agent layer composition; and
spreading the adhesive agent layer composition to obtain the adhesive agent layer, wherein
a particle diameter D₅₀ at which a cumulative volume in a particle size distribution of the silicic anhydride becomes 50% is 0.2 to 9.0 um.

7. The method for producing a poultice according to claim 6, wherein a content of the carboxymethyl cellulose and/or the pharmaceutically acceptable salt thereof in the adhesive agent layer composition and the adhesive agent layer is 1.0 to 4.0% by mass relative to a total mass of the adhesive agent layer in terms of sodium salt.

8. The method for producing a poultice according to claim 6 or 7, wherein a content of the silicic anhydride in the adhesive agent layer composition and the adhesive agent layer is 0.1 to 6.0% by mass relative to the total mass of the adhesive agent layer.

9. The method for producing a poultice according to claim 6 or 7, wherein a mass ratio of the content of the carboxymethyl cellulose and/or the pharmaceutically acceptable salt thereof in terms of sodium salt to the content of the silicic anhydride (content of the carboxymethyl cellulose and/or the pharmaceutically acceptable salt thereof in terms of sodium salt : content of the silicic anhydride) in the adhesive agent layer composition and the adhesive agent layer is 1:2 to 6:0.5.

10. The method for producing a poultice according to claim 6 or 7, wherein the drug is at least one selected from the group consisting of diclofenac, felbinac, flurbiprofen, a salicylic acid ester, indomethacin, ketoprofen, diphenhydramine, loxoprofen, lidocaine, vitamin E, glycyrrhetinic acid, capsicum extract, nonylic acid vanillylamide, l-menthol, dl-camphor, and pharmaceutically acceptable salts thereof.
